# EUROPEAN PATENT APPLICATION

(11) **EP 3 610 855 A1**
(43) Date of publication of application: **19.02.2020**
(21) Application number: 18209343.5
(22) Date of filing: 29.11.2018
(51) Int. Cl.: A61K 9/00, A61K 31/12, A61K 31/352, A61K 31/714, G02B 1/04

(54) **CONTACT LENS WITH FUNCTIONAL COMPONENTS AND PRODUCTS THEREOF**

(30) Priority: 14.08.2018 TW 107128286
(71) Applicant: UNICON Optical Co, LTD., Baoshan Township, Hsinchu County 30075 (TW)
(72) Inventor: ROUT, Bishakh, 300 Hsinchu City (TW); WANG, Chih-Chung, 302 Hsinchu County (TW); HSIEH, Jung-Yuan, 300 Hsinchu City (TW)
(74) Representative: Lang, Christian

(57) **Abstract**

A contact lens with functional components and products thereof are disclosed, wherein at least one bonding agent is added to the polymeric matrix material of the lens body. The bonding agent can effectively integrate with various kinds of functional components to improve the characteristics of the lens body, increase the comfort of wearing the contact lens, and enhance the ability of water retention. While the user wears the contact lens, the contact lens can release the functional components persistently. Therefore, the present invention can relieve irritations caused by dust, dirt, smoke, and/or pollution from the environment.

## Description

This application claims priority for Taiwan patent application no. 107128286 filed on August 14, 2018, the content of which is incorporated by reference in its entirety.

### BACKGROUND OF THE INVENTION

### Field of the Invention

The present invention relates to a technology of contact lenses, particularly to a contact lens with functional components and products thereof, wherein the polymeric matrix material of the contact lens can combine with required functional components and stably release the functional components during usage.

### Description of the Related Art

Contact lenses can replace ordinary glasses to correct problems of vision. Therefore, it is a popular choice for the people suffering visual degradation, such as myopia. Further, "contact lens" also assumes the position of a vogue word. The contact lenses with colored design around the optical area have become the essential fashion accessories in many countries. Some types of contact lenses have the function of correcting eyesight and the function of presenting the user's stylishness simultaneously.

According to materials, contact lenses may be categorized into hard contact lenses and soft contact lenses. The materials usually used to fabricate contact lens include siloxane monomers, poly(2-hydroxyethyl methacrylate (pHEMA), and combinations thereof. Soft contact lenses are usually cheaper and thus popular in the market. However, the materials for soft contact lenses may deprive the cornea of oxygen. Although silicone monomer can overcome the problem, it still confronts many challenges, such depositions attached to the hydrophobic contact lenses. Therefore, contact lenses still have some problems to solve even though the materials thereof have been improved.

One of the problems of using contact lenses is the irritation to eyes after long-term usage. It is said that the contact lens-induced ocular inflammation is related with the following factors: stimulation of contact lenses to ocular cells, oxygen deprivation by lens materials, and discomfort caused by the shape and surface of lenses. Another problem of using contact lenses is ocular dryness. In fact, it is very hard to find a contact lens able to completely solve the problems caused by different factors.

One method of relieving the irritation or discomfort caused by contact lenses is to deliver some functional components to eyes. The selected functional components may benefit different regions of the ocular structure, such alleviating inflammation, relaxing ciliary muscle, and generating cool sensation. Compared with using the conventional eye drops, delivering the functional components to eyes through contact lenses is more favorable to users. Most of the eye drops are lost in the nasolacrimal duct, and the rest is lost in the actions of eyes (such as blinks and eyeball rotations).

Contact lenses can carry the functional components to the ocular structure and stably release the functional components for a longer period of time. Thereby, the functional components would not be lost in various actions of eyes. Accordingly, the present invention proposes a contact lens with functional components and products thereof, wherein at least one bonding agent, such as natural cyclic oligosaccharide or polysaccharide, is dispersed in the contact lens to effectively combine with useful functional components and stably release the functional components.

### SUMMARY OF THE INVENTION

The primary objective of the present invention is to provide a contact lens with functional components and products thereof, wherein at least one bonding agent, such as a cyclic oligosaccharide or polysaccharide, is uniformly distributed in the polymeric matrix material of the contact lens, and wherein the bonding agent can absorb various kinds of functional components and enables the lens body to release the functional components stably for a longer period of time.

Another objective of the present invention is to provide a contact lens with functional components and products thereof, wherein the functional components combine with the bonding agent in the contact lens, whereby the contact lens can stably release the functional components to the ocular environment and relieve various types of discomforts of eyes.

In order to achieve the abovementioned objectives, the present invention proposes a contact lens with functional components, which comprises a lens body and at least one functional component. The lens body is a reaction product of a polymerizable composite. The polymerizable composite includes a polymeric matrix material and at least one bonding agent. The bonding agent is selected from a group including cyclic oligosaccharides, cyclic polysaccharides, polyethylene glycol (PEG), poly vinyl alcohol (PVA), polyacrylamide (PAM), alginate, hyaluronate, micro platelet cellulose (MPC), hydroxypropyl methylcellulose (HPMC), and combinations thereof. The bonding agent is uniformly distributed in the polymeric matrix material. The functional components can be absorbed by the bonding agent distributed in the polymeric matrix material and thus effectively integrated with the lens body. The functional component is selected from a group including vitamins, phytochemicals, growth factors, therapeutic compounds, natural derivatives, bioactive compounds, pH detecting agents, and combinations thereof. While a user is wearing the contact lens, the lens body can release the functional components stably.

In the contact lens with functional components of the present invention, the proportion of the functional components in the lens body is preferably 0.5-5wt%.

The present invention also proposes a product of a contact lens with functional components. The product comprises a lens body and a package. The lens body is a reaction product of a polymerizable composite. The polymerizable composite includes a polymeric matrix material and at least one bonding agent. The bonding agent is selected from a group including cyclic oligosaccharides, cyclic polysaccharides, polyethylene glycol (PEG), poly vinyl alcohol (PVA), polyacrylamide (PAM), alginate, hyaluronate, micro platelet cellulose (MPC), hydroxypropyl methylcellulose (HPMC), and combinations thereof. The bonding agent is uniformly distributed in the polymeric matrix material. The package includes a buffer solution where the lens body is to be soaked. The buffer solution includes at least one functional component. The functional component can be absorbed by the bonding agent distributed in the polymeric matrix material and thus effectively integrated with the lens body. The functional component is selected from a group including vitamins, phytochemicals, growth factors, therapeutic compounds, natural derivatives, bioactive compounds, pH detecting agents, and combinations thereof. While a user takes the lens body from the buffer solution of the package and wears the contact lens, the lens body can stably release the functional components to the ocular region and relieve various types of discomfort of eyes.

In the product of the contact lens with functional components of the present invention, the proportion of the functional components in the buffer solution is preferably 0.5-5w/v%.

In the cases where a cyclic oligosaccharide or a cyclic polysaccharide is used as the bonding agent in the contact lens with functional components and products thereof of the present invention, the cyclic oligosaccharide or cyclic polysaccharide may cooperate with various functional components to form complexes. As the formation of the complexes is a reversible phenomenon, the functional components can be stably released from the complexes. On the other hand, while the lens body of the present invention is placed in a region having high concentration of functional components, the functional components may cooperate with the cyclic oligosaccharide or polysaccharide in the polymeric matrix material of the lens body to form complexes. Once the lens body is placed in a region having lower concentration of functional components (such as the ocular region of the user), the lens body will stably release the functional components thereof. Thereby, the functional components may work to contribute their functionalities.

Below, embodiments will be described in detail to make easily understood the objectives, technical contents, characteristics, and accomplishments of the present invention.

### BRIEF DESCRIPTION OF THE DRAWINGS

- Fig.1: is a diagram schematically showing a product of a contact lens with functional components according to one embodiment of the present invention;
- Fig.2: is a diagram showing an experimental result of the accumulated amount of Vitamin B12 released by a HEMA contact lens of 38% water content according to one embodiment of the present invention;
- Fig.3: is a diagram showing an experimental result of the accumulated amount of Vitamin B12 released by a contact lens made up of organosilicon hydrogel and containing 43% water by weight according to one embodiment of the present invention;
- Fig.4: is a diagram showing an experimental result of the accumulated amount of curcumin released by a HEMA contact lens of 38% water content according to one embodiment of the present invention;
- Fig.5: is a diagram showing an experimental result of the accumulated amount of quercetin released by a HEMA contact lens of 38% water content according to one embodiment of the present invention; and
- Fig.6: is a diagram showing an experimental result of the accumulated amount of xanthophyll released by a HEMA contact lens of 38% water content according to one embodiment of the present invention.

### DETAILED DESCRIPTION OF THE INVENTION

The present invention discloses a contact lens with functional components, which comprises a lens body and at least one functional component. The lens body is a reaction product of a polymerizable composite. The polymerizable composite includes a polymeric matrix material and at least one bonding agent uniformly distributed in the polymeric matrix material. The functional components can be absorbed by the bonding agent distributed in the polymeric matrix material and thus effectively integrated with the lens body.

In the present invention, the material of the contact lens is a polymeric film produced in a light polymerization method or a thermal polymerization method. The contact lens is fabricated in a lathe-cutting technology, a cast-molding technology, a spin-coating technology, a UV curing technology, and/or a heat curing technology. For the persons skilled in the art, it is well known: the materials of the polymeric matrix, which is used to produce the lens body of the contact lens, may include hydrophilic monomers (such as poly-2(hydroxyethyl methacrylates (pHEMA)), hydrophobic monomers (such as Silicones), or mixtures thereof. The mixtures of the monomers may contain other materials or components having special functions, such as a photo initiator, a thermal initiator, and/or an additive able to increase the hydrophilicity of the contact lens. In order to make a contact lens able to release the functional components, the bonding agent must be uniformly distributed in the mixture of monomers. The proportion of the bonding agent is dependent on the solubility thereof. For example, 1-5%(v/v) of the solution of the bonding agent is added to the mixture of monomers, and then agitated and degassed for fabricating lenses. In the present invention, the bonding agents are referred to agents able to absorb and store required functional components in various ways. The bonding agent absorbs and stores functional components via a complexation effect, a matrix effect, an ionic attraction effect, or combinations thereof. The bonding agent may be but is not limited to be selected from a group including cyclic oligosaccharides, cyclic polysaccharides, polyethylene glycol (PEG), poly vinyl alcohol (PVA), polyacrylamide (PAM), alginate, hyaluronate, micro platelet cellulose (MPC), hydroxypropyl methylcellulose (HPMC), and combinations thereof.

In the present invention, addition of the bonding agent to the polymeric matrix material not only modifies the properties of the material of the lens body, such as decreases friction and increases softness, but also improves the comfort of wearing the lens body. Further, addition of the bonding agent to the polymeric matrix material enhances the ability of water retention. The combination of water and functional components enables persistent release of the functional components.

The cyclic oligosaccharides or polysaccharides are compounds formed via cyclically combining carbohydrate molecules. The compounds assume different forms, depending on the kinds of carbohydrate molecules cyclically combined. The compounds may be but is not limited to be selected from a group including cyclodextrins, cyclo isomalto-oligosamlharides, alternans, cyclic (1→2)-beta-D-glucans (cyclosophorans), cycloraminarans, and cyclofructans. In the present invention, the cyclodextrin may be α-cyclodextrin, β-cyclodextrin, γ-cyclodextrin, or a combination thereof. The special cyclic structures of the compounds enable them to cooperate with other materials to form complexes, which are normally called guest-host compounds or clathrates. The guest compound is normally enclosed by the host compound (the cyclic oligosaccharide or cyclic polysaccharide).

The cyclic oligosaccharide or cyclic polysaccharide may be chemically modified via reacting with special alkyl radicals, whereby to improve the ability of combining with hydrophobic or hydrophilic components. Further, the cyclic oligosaccharide or cyclic polysaccharide may be coupled to nanocarriers to form cyclic oligosaccharide- or cyclic polysaccharide-carrying nanoparticles, and the nanoparticles are dispersed in the polymeric matrix material of the lens body. The cyclic oligosaccharide- or cyclic polysaccharide-carrying nanoparticle is a nanocarrier having smaller cyclic oligosaccharide or cyclic polysaccharide molecules in the interior thereof. Such a structure can enhance the combination of the lens body and the functional components through the cyclic oligosaccharide or cyclic polysaccharide. The nanocarriers suitable for the present invention may be selected from a group including lipid nanoparticles, liposomes, micelles, and surfactant aggregates.

Herein is provided an embodiment of fabricating the lens body, and the steps thereof are described below. Firstly, add 2g of methacrylic acid (MAA) to 50g of hydroxyethyl methacrylate to form a first mixture. Next, add 1g of polymerization initiator 2-Hydroxy-2-methyl-1-phenyl-propan-1-one (Ciba Darocur 1173) and 1g of ethylene glycol dimethacrylate (EGDMA) to the first mixture to acquire a first monomer mixture. Next, agitate the first monomer mixture at a temperature of 35°C and a speed of 200 rpm for 1 hour. At the same time, dissolve 25mg of α-cyclodextrin in 1 ml of deionized water to form a first suspension. Next, agitate the first suspension at a temperature of 45°C and a speed of 250 rpm for 1 hour. After the α-cyclodextrin is fully dissolved, add the α-cyclodextrin solution to the abovementioned first monomer mixture to form a second monomer mixture. For example, 5ml of α-cyclodextrin solution is added to 995ml of monomer mixture of HEMA-MAA-Darocur 1173-EGDMA to form the second monomer mixture. Next, agitate the second monomer mixture until the second monomer mixture becomes a clear solution. Next, pour the clear solution into a mold. Next, use an ultraviolet light with a wavelength of 310-370nm and an energy density of 28-32mW/cm² to cure the solution for 40 minutes. Next, use appropriate tools and appropriate solvents to demold the lens from the mold. Then, rinse the lens for a sufficient period of time to remove monomers and other agents, which do not participate in reaction.

The present invention also provides an embodiment for modifying cyclic oligosaccharides or cyclic polysaccharides, which comprises steps: dissolving 22mg of β-cyclodextrin in 1 ml of deionized water via agitation at a temperature of 45°C and a speed of 250 rpm for 1 hour to form a solution; adding 0.1ml of chloracetic acid to the solution to obtain a mixture solution; heating the mixture solution to a temperature of 60°C, and agitating the mixture solution at a speed of 300 rpm for 4 hours; vacuum-drying the mixture solution in an oven at a temperature of 70°C until fine white powder is acquired. The powder is a chemically-modified cyclic oligosaccharide.

The present invention also provides an embodiment for producing cyclic oligosaccharide- or cyclic polysaccharide-carrying nanoparticles, which comprises steps: using isopropyl alcohol, octadecylamine, and glyceryl dibehenate to fabricate lipid nanoparticles; after drying, adding deionized water to lipid nanoparticles and mixing them appropriately to obtain a solution of lipid nanoparticles; adding the abovementioned cyclodextrin or the combination thereof to the solution of lipid nanoparticles; agitating the solution, and mixing the solution with the abovementioned monomer mixture to acquire appropriate mixture.

In the present invention, the functional component is referred to a substance beneficial to ocular regions. The functional components used by the present invention may be but is not limited to be selected from a group including vitamins, phytochemicals, growth factors, therapeutic compounds, natural derivatives, bioactive compounds, pH detecting agents, and combinations thereof. In detail, the vitamin may be selected from a group including Vitamin B6, Vitamin B12, Vitamin C, and Vitamin E; the phytochemical may be selected from a group including chlorophyll, bilberry extract, curcumin, quercetin, xanthophyll, and Chinese angelica extract; the growth factor may be selected from a group including epidermal growth factor (EGF), vascular endothelial growth factor (VEGF), platelet-derived growth factor (PDGF), retinoic acid, and butylidenephthalide; the therapeutic compound may be selected from a group including cyclosporine A, erythromycin, ciprofloxacin, gatifloxacin, ofloxacin, polymyxin B, besifloxacin, moxifloxacin, gentamicin, tobramycin, neomycin, natamycin, luconazole, voriconazole, antihistamines, dexamethasone, timolol maleate, puerarin, tetrahydrozoline hydrochloride, and chlorpheniramine maleate; the natural derivative may be selected from a group including genistein, myriocin, mangiferin, baicalin, resina draconis, tetrandrine, lycopene, anthocyanin and combinations thereof; the bioactive compound may be mitochondria extracted from stem cells. The other functional components may include the pH detecting agent, which may be selected from a group including anthocyanins and flavins. The quantity of the functional component used in the lens body is dependent on the type thereof. In general, the proportion of the functional components in the lens body is about 0.5-5wt%. The combination of the functional components and the bonding agent, such as the cyclic oligosaccharide or cyclic polysaccharide, may be achieved via soaking the lens body in a region having high concentration of functional components.

In the present invention, the functional components provide at least one benefit to the ocular region. The benefits may include but are not limited to be providing comfort (by Vitamin B6), relaxing the ciliary muscle (by Vitamin B12), moisturizing eyes (by Vitamins B6 and/or B12), relieving inflammation (by Vitamin B12 and/or curcumin), reducing free radical-induced damage (by quercetin, xanthophyll, chlorophyll, and/or bilberry extract), relieving the inflammation or redness caused by anoxia or xerophthalmia (by Vitamin B12, curcumin and/or quercetin), decreasing the probability of age-related macular degeneration (by xanthophyll), lowering intraocular pressure (by timolol maleate and/or dexamethasone), inhibiting fungal/bacterial infection (by erythromycin, ciprofloxacin, gatifloxacin, ofloxacin, polymyxin B, besifloxacin, moxifloxacin, gentamicin, tobramycin, neomycin, natamycin, fluconazole, and/or voriconazole), inhibiting allergy (by antihistamines), activating ocular cell regeneration (by EGF, VEGF, PDGF, retinoic acid, and/or butylidenephthalide), and increasing the amount of healthy ocular tissue (by mitochondria extracted from stem cells). The other functional components, such as anthocyanins and flavins, may function as pH detecting agents to detect the pH value of tears while they contact tears. The variation of the pH value may be used to diagnose diseases.

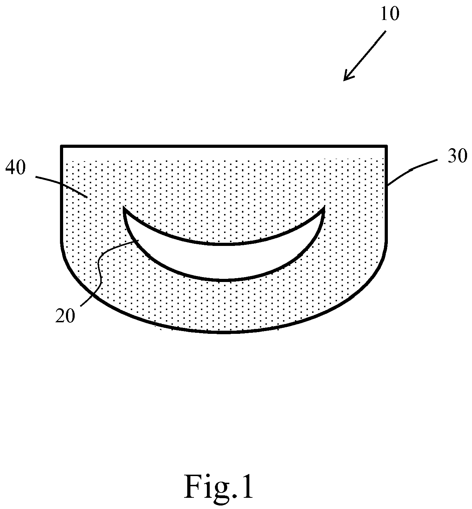

In order to achieve the combination of required functional components and the lens body, the lens body may be soaked in a region having high concentration of functional components. The product of the contact lens with functional components of the present invention is normally used to realize the combination of the contact lens and the functional components in practical application. Refer to Fig.1. The present invention also discloses a product 10 of the contact lens with functional components. The product 10 comprises a lens body 20 and a package 30. The lens body 20 is a reaction product of a polymerizable composite. The polymerizable composite includes a polymeric matrix material and at least one bonding agent uniformly distributed in the polymeric matrix material. The lens body 20 is soaked in a germ-free buffer solution 40 stored in a polypropylene container. The buffer solution 40 has functional components. The functional components may be absorbed by the bonding agent and thus combined with the lens body 20.

The buffer solution 40 used in the package 30 is defined to be a contact-lens pH-control solution stored in a polypropylene container. In the present invention, the buffer solution 40 is prepared from one of the following buffer systems: phosphate, borate, tris(hydroxymethyl)aminomethane (TRIS), morpholinopropanesulfonate (MOPS), and 4-(2-Hydroxyethyl)piperazine-1-ethanesulfonic acid (HEPES). One or more functional components are added to the buffer solution 40. The proportion of the functional components in the buffer solution 40 is about 0.5-5% (w/v). For example, 8g of sodium chloride, 2.5g of dihydrate of disodium phosphate, and 0.4g of dihydrate of monosodium phosphate are added to 1000ml of water to prepare the buffer solution 40. 100mg of Vitamin B12 is added to the buffer solution 40, and the buffer solution 40 is agitated. Then, the lens body 20 is stored in the buffer solution 40 contained in the container. A wet heating method or a γ radiation method is used for sterilization. Thus is obtained a product 10 of the contact lens with functional components.

In order to realize the objectives of the present invention, the components of the buffer solution 40 may be modified corresponding to the functional components, whereby to make the buffer solution 40 have the required pH value and the required osmotic pressure. For example, the buffer solution 40 has an appropriate concentration of Vitamin B12. Naturally, the other functional components functioning like Vitamin B12 may also be added to the buffer solution 40.

The product 10 of the contact lens with functional components of the present invention may contribute benefits to eyes. For example, the contact lens combining with antibiotics may provide anti-bacterial function to the eyes, in addition to the eyesight correcting effect and the cosmetic effect (of the colored contact lenses).

Below is provided an embodiment to fully demonstrate how the present invention fabricates the product of the contact lens with functional components.

Firstly, in Step S100, take hydroxyethyl methacrylate (HEMA) weighing 50g, and add 2g of methyl acrylic acid (MAA) to HEMA to form a first mixture.

Next, in Step S110, add 1g of polymerization initiator 2-Hydroxy-2-methyl-1-phenyl-propan-1-one (Ciba ™ Darocur ™ 1173) and 1g of ethylene glycol dimethacrylate (EGDMA) to the first mixture to acquire a first monomer mixture, and agitate the first monomer mixture at a temperature of 35°C and a speed of 200 rpm for 1 hour.

Next, in Step S120, dissolve α-cyclodextrin weighing 25mg in 1 ml of deionized water to form a first suspension, and agitate the first suspension at a temperature of 45°C and a speed of 250 rpm for 1 hour. After the α-cyclodextrin is fully dissolved, add the α-cyclodextrin solution to the abovementioned first monomer mixture to form a second monomer mixture. For example, 50ml of α-cyclodextrin solution is added to 950ml of monomer mixture of HEMA-MAA-Darocur 1173-EGDMA to form the second monomer mixture, and the second monomer mixture is agitated until the second monomer mixture becomes a clear solution.

Next, in Step S130, pour the clear solution into a mold, and use an ultraviolet light with a wavelength of 310-370nm and an energy density of 28-32mW/cm² to cure the solution for 40 minutes.

Next, in Step S140, after curing is done, soak the semi-product of the lens body in a 20% ethanol solution at a temperature of 80°C for 30 minutes, and rinse the lens body with deionized water at a temperature of 80°C for 45 minutes to remove chemical compounds, which do not participate in reaction.

In Step S150, add 8g of sodium chloride, 2.5g of dihydrate of disodium phosphate, and 0.4g of dihydrate of monosodium phosphate to 1000ml of water to prepare the buffer solution, add Vitamin B12 to the buffer solution, and agitate the buffer solution; use the buffer solution in the storage of the lens body having been rinsed with water, wherein the lens body and the buffer solution are together placed inside a polypropylene (PP) container.

Then, in Step S160, use a wet heating method or a γ radiation method to disinfect the PP container carrying the lens body. Thus, the contact lens with functional components is ready for being used by the user.

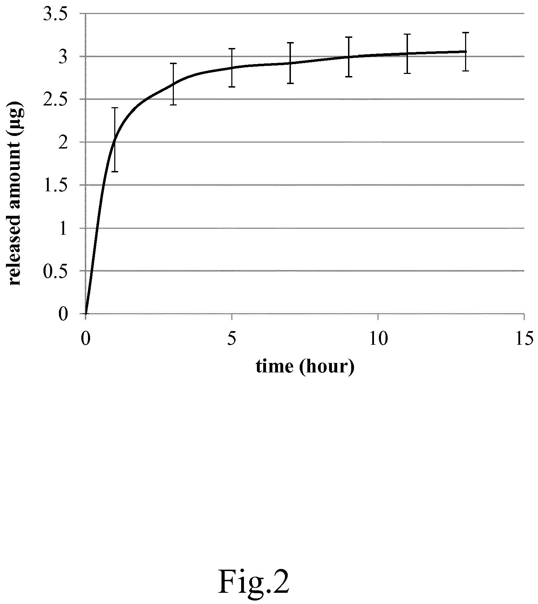

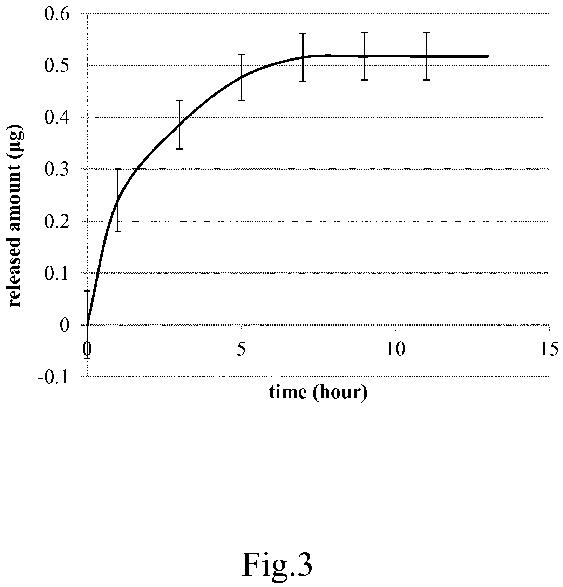

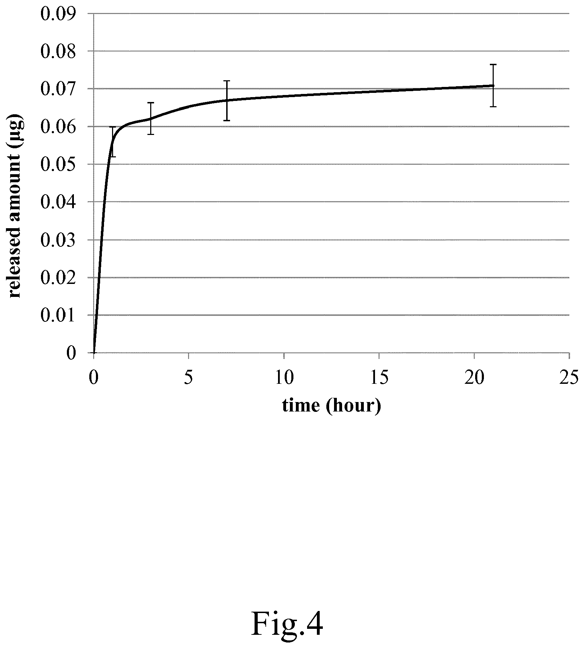

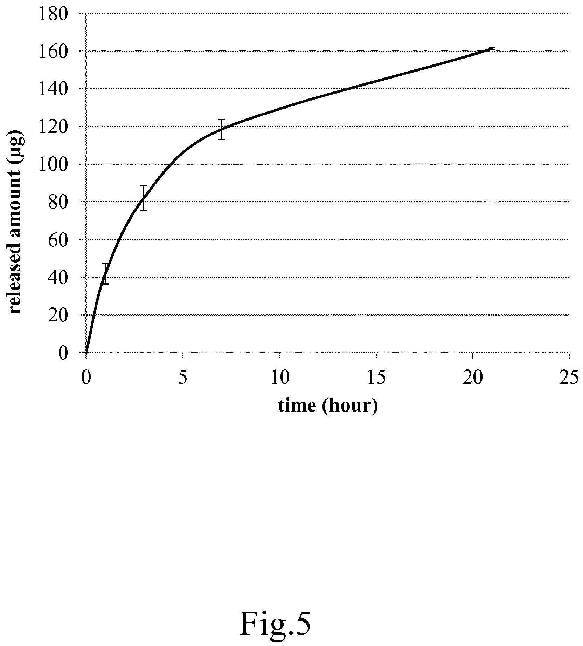

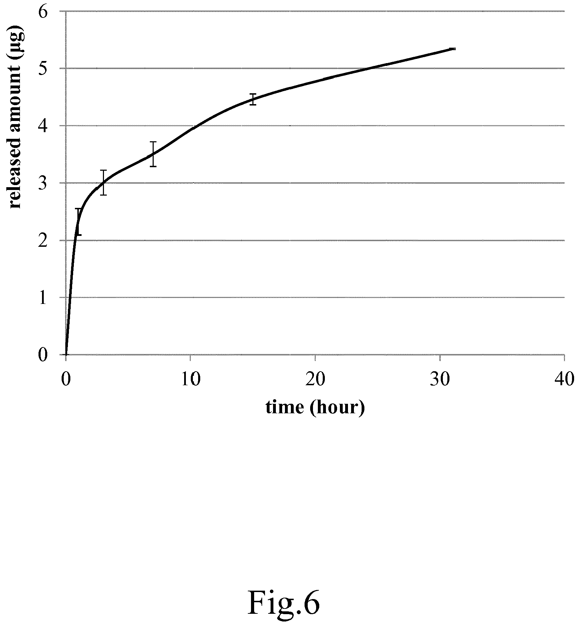

Further, the present invention uses high performance liquid chromatography to detect accumulation of the released functional components of the contact lens with increasing time. It is learned from Fig.2: the contact lens of the present invention, which is a HEMA lens made up of 38% water by weight and a cyclic oligosaccharide or cyclic polysaccharide, can persistently release Vitamin B12 for a longer period of time (more than 12 hours). It is learned from Fig.3: the contact lens of the present invention, which is an organosilicon hydrogel contact lens made up of 43% water by weight and a cyclic oligosaccharide or cyclic polysaccharide, can persistently release Vitamin B12 for a longer period of time (more than 12 hours). It is learned from Fig.4: the contact lens of the present invention, which contains HEMA and 38% water by weight and a cyclic oligosaccharide or cyclic polysaccharide, can persistently release curcumin for a longer period of time (more than 20 hours). It is learned from Fig.5: the contact lens of the present invention, which contains HEMA and 38% water by weight and a cyclic oligosaccharide or cyclic polysaccharide, can persistently release quercetin in a longer time interval (more than 20 hours). It is learned from Fig.6: the contact lens of the present invention, which is made from HEMA and 38% water by weight, and a cyclic oligosaccharide or cyclic polysaccharide, can persistently release xanthophyll for a longer period of time (more than 30 hours).

In conclusion, the present invention discloses a contact lens with functional components and products thereof, wherein at least one bonding agent is added to the polymeric matrix material of the lens body, and wherein the bonding agent integrates with various kinds of functional components and enables the lens body to release the functional components stably for a longer period of time. While the user wears the contact lens with functional components, the functional components is persistently released by the contact lens to the ocular region, performing their functions and benefiting the eyes. Therefore, the present invention can relieve irritations caused by dust, dirt, smoke, and/or pollution from the environment.

The embodiments described above are only to exemplify the present invention but not to limit the scope of the present invention. Any equivalent modification or variation according to the characteristic or spirit of the present invention is to be also included by the scope of the present invention.

## Claims

1. A contact lens with functional components, comprising
a lens body being a reaction product of a polymerizable composite, wherein said polymerizable composite includes a polymeric matrix material and at least one bonding agent; said bonding agent is uniformly distributed in said polymeric matrix material; said bonding agent is selected from a group consisting of cyclic oligosaccharides, cyclic polysaccharides, polyethylene glycol (PEG), poly vinyl alcohol (PVA), polyacrylamide (PAM), alginate, hyaluronate, micro platelet cellulose (MPC), hydroxypropyl methylcellulose (HPMC), and combinations thereof; and
at least one functional component absorbed by said bonding agent and integrated with said lens body, wherein said functional component is selected from a group consisting of vitamins, phytochemicals, growth factors, therapeutic compounds, natural derivatives, bioactive compounds, pH detecting agents, and combinations thereof.

2. The contact lens with functional components according to claim 1, wherein said cyclic oligosaccharide or said cyclic polysaccharide is coupled to nanocarriers to form cyclic oligosaccharide- or cyclic polysaccharide-carrying nanoparticles.

3. The contact lens with functional components according to claim 2, wherein said nanocarrier is selected from a group consisting of lipid nanoparticles, liposomes, micelles, and surfactant aggregates.

4. The contact lens with functional components according to claim 1, wherein said vitamin is selected from a group consisting of Vitamin B6, Vitamin B12, Vitamin C, and Vitamin E; said phytochemical is selected from a group consisting of chlorophyll, bilberry extract, curcumin, quercetin, xanthophyll, and Chinese angelica extract; said growth factor is selected from a group consisting of epidermal growth factor (EGF), vascular endothelial growth factor (VEGF), platelet-derived growth factor (PDGF), retinoic acid, and butylidenephthalide; said therapeutic compound is selected from a group consisting of cyclosporine A, erythromycin, ciprofloxacin, gatifloxacin, ofloxacin, polymyxin B, besifloxacin, moxifloxacin, gentamicin, tobramycin, neomycin, natamycin, luconazole, voriconazole, antihistamines, dexamethasone, timolol maleate, puerarin, tetrahydrozoline hydrochloride, and chlorpheniramine maleate; said natural derivative is selected from a group consisting of genistein, myriocin, mangiferin, baicalin, resina draconis, tetrandrine, lycopene, anthocyanin and combinations thereof; said bioactive compound is mitochondria extracted from stem cells; said pH detecting agent is selected from a group consisting of anthocyanins and flavins.

5. The contact lens with functional components according to claim 1, wherein a proportion of said functional components in said lens body is 0.5-5wt%.

6. The contact lens with functional components according to claim 1, wherein said contact lens is fabricated in a lathe-cutting technology, a cast-molding technology, a spin-coating technology, a UV curing technology, or a heat curing technology.

7. A product of a contact lens with functional components, comprising
a lens body being a reaction product of a polymerizable composite, wherein said polymerizable composite includes a polymeric matrix material and at least one bonding agent; said bonding agent is uniformly distributed in said polymeric matrix material; said bonding agent is selected from a group consisting of cyclic oligosaccharides, cyclic polysaccharides, polyethylene glycol (PEG), poly vinyl alcohol (PVA), polyacrylamide (PAM), alginate, hyaluronate, micro platelet cellulose (MPC), hydroxypropyl methylcellulose (HPMC), and combinations thereof; and
a package consisting of a buffer solution where said lens body is to be soaked, wherein said buffer solution further includes at least one functional component; said functional component is absorbed by said bonding agent and integrated with said lens body; said functional component is selected from a group consisting of vitamins, phytochemicals, growth factors, therapeutic compounds, natural derivatives, bioactive compounds, pH detecting agents, and combinations thereof.

8. The contact lens according to claim 1 or the product of a contact lens with functional components according to 7, wherein said cyclic oligosaccharide or said cyclic polysaccharide is selected from a group consisting of cyclodextrins, cyclo isomalto-oligosamlharides, alternans, cyclic (1→2)-beta-D-glucans (cyclosophorans), cycloraminarans, and cyclofructans.

9. The contact lens or the product of a contact lens with functional components according to claim 8, wherein said cyclodextrin is selected from a group consisting of α-cyclodextrin, β-cyclodextrin, γ-cyclodextrin, and combinations thereof.

10. The contact lens according to claim 1 or the product of a contact lens with functional components according to 7, wherein said cyclic oligosaccharide or said cyclic polysaccharide is chemically modified via reacting with special alkyl radicals.

11. The product of a contact lens with functional components according to claim 7, wherein said cyclic oligosaccharide or said cyclic polysaccharide is coupled to nanocarriers to form cyclic oligosaccharide- or cyclic polysaccharide-carrying nanoparticles.

12. The product of a contact lens with functional components according to claim 11, wherein said nanocarrier is selected from a group consisting of lipid nanoparticles, liposomes, micelles, and surfactant aggregates.

13. The product of a contact lens with functional components according to claim 7, wherein said vitamin is selected from a group consisting of Vitamin B6, Vitamin B12, Vitamin C, and Vitamin E; said phytochemical is selected from a group consisting of chlorophyll, bilberry extract, curcumin, quercetin, xanthophyll, and Chinese angelica extract; said growth factor is selected from a group consisting of epidermal growth factor (EGF), vascular endothelial growth factor (VEGF), platelet-derived growth factor (PDGF), retinoic acid, and butylidenephthalide; said therapeutic compound is selected from a group consisting of cyclosporine A, erythromycin, ciprofloxacin, gatifloxacin, ofloxacin, polymyxin B, besifloxacin, moxifloxacin, gentamicin, tobramycin, neomycin, natamycin, luconazole, voriconazole, antihistamines, dexamethasone, timolol maleate, puerarin, tetrahydrozoline hydrochloride, and chlorpheniramine maleate; said natural derivative is selected from a group consisting of genistein, myriocin, mangiferin, baicalin, resina draconis, tetrandrine, lycopene, anthocyanin and combinations thereof; said bioactive compound is mitochondria extracted from stem cells; said pH detecting agent is selected from a group consisting of anthocyanins and flavins.

14. The product of a contact lens with functional components according to claim 7, wherein a proportion of said functional components in said buffer solution is 0.5-5 w/v %.

15. The product of a contact lens with functional components according to claim 7, wherein said contact lens is fabricated in a lathe-cutting technology, a cast-molding technology, a spin-coating technology, a UV curing technology, or a heat curing technology.
